(19) Europäisches Patentamt / European Patent Office / Office européen des brevets

(11) **EP 3 040 044 B1**

(12) **EUROPEAN PATENT SPECIFICATION**

(45) Date of publication and mention
of the grant of the patent:
**25.08.2021 Bulletin 2021/34**

(51) Int Cl.:
*A61B 18/14* *(2006.01)*    *A61B 5/00* *(2006.01)*
*A61B 34/20* *(2016.01)*    *A61B 18/00* *(2006.01)*
*A61B 17/00* *(2006.01)*

(21) Application number: **15202361.0**

(22) Date of filing: **23.12.2015**

(54) **SPECTRAL SENSING OF ABLATION**

SPEKTRALE ABLATIONSMESSUNG

DÉTECTION SPECTRALE DE L'ABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.12.2014 US 201414585135**

(43) Date of publication of application:
**06.07.2016 Bulletin 2016/27**

(73) Proprietor: **Biosense Webster (Israel) Ltd.
2066717 Yokneam (IL)**

(72) Inventors:
• **GOVARI, Assaf
2066717 Yokneam (IL)**

• **BEECKLER, Christopher Thomas
Irwindale, CA California 91706 (US)**
• **KEYES, Joseph Thomas
Irwindale, CA California 91706 (US)**
• **GLINER, Vadim
2066717 Yokneam (IL)**

(74) Representative: **Carpmaels & Ransford LLP
One Southampton Row
London WC1B 5HA (GB)**

(56) References cited:
**EP-A1- 2 742 892      WO-A2-2012/131577
JP-A- 2012 239 848      US-A1- 2009 054 908
US-A1- 2012 265 184**

## Description

## FIELD OF THE INVENTION

The present invention relates generally to invasive medical devices and particularly to assessing ablation of tissue within the body.

## BACKGROUND

Minimally-invasive intracardiac ablation is the treatment of choice for various types of arrhythmias. To perform such treatment, the physician typically inserts a catheter through the vascular system into the heart, brings the distal end of the catheter into contact with myocardial tissue in areas of abnormal electrical activity, and then energizes one or more electrodes at or near the distal end in order to create tissue necrosis.

Various methods for monitoring ablation treatments are known in the art. For example, U.S. Patent 7,001,383, describes real-time monitoring and mapping of ablation lesion formation in the heart. The disclosed method includes applying a local treatment to the heart at a plurality of sites designated for ablation. At each respective site, a parameter is sensed that is indicative of a level of ablation at the site. The method preferably includes displaying a map of the heart, and designating, on the map, during the ablation procedure, indications of the respective levels of ablation at the sites, responsive to the respective sensed parameters.

U.S. Patent Application Publication 2014/0171936, describes a catheter having an irrigated tip with temperature sensors and an optical fiber array. The catheter comprises an insertion tube having a distal end configured for insertion into proximity with tissue in a body of a patient and containing a lumen having an electrical conductor for conveying electrical energy to the tissue. A conductive cap is attached to the distal end of the insertion tube and is coupled electrically to the electrical conductor. A multiplicity of optical fibers are contained within the insertion tube, each fiber terminating in proximity to the outer surface of the cap, and being configured to convey optical radiation to and from the tissue while the electrical energy is being conveyed to the tissue.

U.S. Patent 8,147,484 describes a system and method that enable real-time optical measurements of tissue reflection spectral characteristics while performing ablation. The method involves the radiation of tissue and recapturing of light from the tissue to monitor changes in the reflected optical intensity as an indicator of steam formation in the tissue for prevention of steam pop. The system includes a catheter adapted to collect light reflected from tissue undergoing ablation, a detection component that identifies and separates constituent wavelengths of collected light, a quantification apparatus for generating measured light intensity data for the collected light, and a processor that analyzes the measured light intensity data in relation to time. A measured reflectance spectral intensity (MRSI) versus time is analyzed, wherein observation is made as to whether the MRSI initially increases in a specified time period followed by a decrease at a specified rate in the MRSI. If there is no decrease in the MRSI, then delivery of ablation energy to tissue continues. However, if there is a decrease in the MRSI within a specified time and at a specified rate, then the method infers the formation of a steam pocket and decreases or discontinues the delivery of ablative energy to tissue.

US2012/0265184A1 describes an ablation catheter comprising a collection optical element disposed in a distal portion, the collection optical element being light-transmissive to collect one or more of returned, backscattered or newly excited light from the targeted tissue region. EP2742892A1 describes a probe including at least one optical sensing unit, which is disposed along the distal segment and includes first and second radiation sources, and an optical sensor configured to receive the optical radiation in first and second wavelength bands.

## SUMMARY

Embodiments of the present invention that are described hereinbelow provide apparatus that can be used in assessing tissue undergoing an ablation procedure according to appended claim 1. Preferred embodiments are disclosed in the dependent claims.

There is provided, in accordance with an embodiment of the present invention, medical apparatus, including an invasive probe, which is configured to be inserted into a body of a living subject, to direct light at a plurality of different wavelengths toward a treatment site within the body and to receive the light scattered from the site. An optical module is coupled to the invasive probe so as to make, at a first stage in ablation of tissue at the treatment site, first measurements of scattered light intensities from the site at the plurality of different wavelengths, and to make, at a second stage in the ablation of the tissue, subsequent to the first stage, second measurements of the scattered light intensities from the site at the plurality of different wavelengths. A processor is configured to assess a progress of the ablation by computing different, respective measures of change in the scattered light intensities at the different wavelengths occurring between the first and second measurements, and comparing the respective measures.

The present invention will be more fully understood from the following detailed description of the embodiments thereof, taken together with the drawings in which:

## BRIEF DESCRIPTION OF THE DRAWINGS

Fig. 1 is a schematic pictorial illustration of a system for intracardiac ablation, in accordance with an embodiment of the present invention;

Fig. 2 is a schematic detail view of a catheter in contact with myocardial tissue during an ablation procedure, in accordance with an embodiment of the present invention;

Fig. 3 is a schematic end view of the distal tip of a catheter with optical sensing capabilities, in accordance with an embodiment of the present invention;

Fig. 4 is a block diagram that schematically illustrates an optical module, in accordance with an embodiment of the present invention;

Figs. 5 and 6 are schematic spectral plots of scattered light intensity from ablation sites at successive stages in ablation procedures at the sites, in accordance with an embodiment of the present invention; and

Fig. 7 is a schematic plot comparing ablation lesion depth to a ratio of scattered light intensities over multiple lesions, in accordance with an embodiment of the present invention.

## DETAILED DESCRIPTION OF EMBODIMENTS

One of the challenges in ablation of myocardial tissue is to know when to stop the treatment at a given site: Applying too little energy may result in creation of only a superficial lesion, which does not achieve the desired therapeutic purpose, whereas applying too much energy can cause excessive tissue damage and even perforation. It is therefore desirable to assess the size (and particularly the depth) of the lesion that has been created at any point during the ablation procedure, and thus to terminate the procedure when it has achieved the desired lesion size.

The inventors have discovered in this regard that certain optical spectral properties of myocardial tissue change during ablation, and that these properties give a good indication of lesion size. Specifically, the inventors have found that the intensity of scattered light changes by different degrees at different wavelengths during ablation, particularly at selected wavelengths in the red and near infrared ranges. The relation between the relative scattering changes at different wavelengths, which may be expressed as a quotient of the ratios of pre- and post-ablation scattering intensities measured at the different wavelengths, correlates well with lesion depth.

Thus, in the embodiments that are described hereinbelow, an invasive probe is applied to a site in the body at which tissue is to be ablated. The probe is used to make measurements of scattered light intensities from the site at a number of different wavelengths at different stages in the ablation process. A processor computes different, respective measures of change in the scattered light intensities at the different wavelengths that occur between the first and second measurements, and compares the respective measures in order to assess the progress of the ablation. Such measurements may be made and compared, for example, before, during, and at the conclusion of the process, and provide an estimate of the lesion size, and specifically the lesion depth, at each stage.

The embodiments described below relate to ablation of myocardial tissue using a probe in the form of a catheter, which applies energy (such as electrical energy) in order to ablate the tissue. The catheter contains one or more optical fibers, with optical ports at the distal end of the catheter through which light at different wavelengths is directed toward the tissue and scattered light is received, for purposes of spectroscopic comparison. Alternatively, however, the principles of the present invention may be applied using probes and optical receivers and transmitters of other types, in ablation of both myocardial tissue and other types of tissue, using any suitable ablation technique that is known in the art.

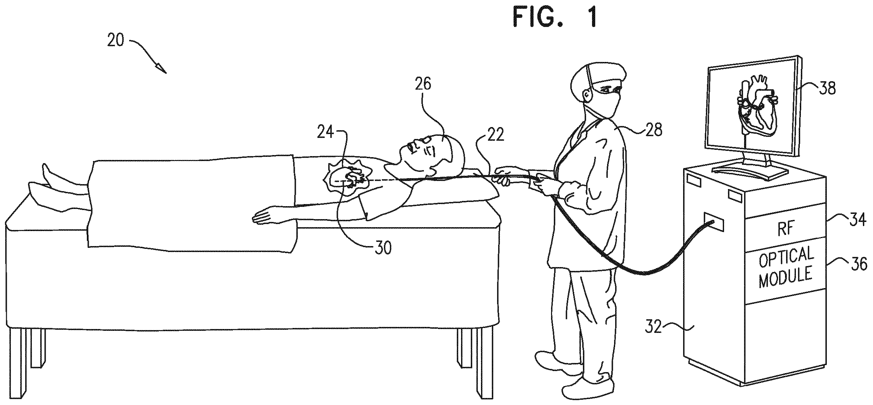

Fig. 1 is a schematic pictorial illustration of a system 20 for cardiac ablation treatment, in accordance with an embodiment of the present invention. An operator 28 (such as an interventional cardiologist) inserts a catheter 22 via the vascular system of a patient 26 into a chamber of the patient's heart 24. For example, to treat atrial fibrillation, the operator may advance the catheter into the left atrium and bring a distal end 30 of the catheter into contact with myocardial tissue that is to be ablated.

Catheter 22 is connected at its proximal end to a console 32, which is controlled by operator 28 to apply and monitor the desired treatment. Console 32 in this embodiment comprises a radio frequency (RF) energy generator 34, which supplies electrical power via catheter 22 to distal end 30 in order to ablate the target tissue. An optical module 40 provides optical radiation, typically from one or more light sources, which may comprise lasers, incandescent lamps, arc lamps, or light emitting diodes (LEDs), for transmission from distal end 30 to the target tissue. Module 40 receives and analyzes optical radiation returning from the target tissue and acquired at the distal end, as described below. On the basis of these results, console 32 may control the power applied by RF energy generator 34, as well as other aspects of the ablation procedure, either automatically or in response to inputs by operator 28. For this latter purpose, console 32 typically presents the relevant measurement results on a display 38.

Console 32 may also receive and track signals from catheter 22 relating to parameters such as the location and of distal end 30 and the force exerted by the distal end on the tissue. An irrigation pump in console 32 typically supplies a cooling fluid, such as saline solution, through catheter 22 to irrigate distal end 30. System 20 may be based in part on the CARTO system, produced by Biosense Webster Inc. (Diamond Bar, California), which provides these sorts of facilities to support navigation and control of catheter 22. These optional features of system 20, however, are beyond the scope of the present description and are omitted from the figures for the sake of simplicity.

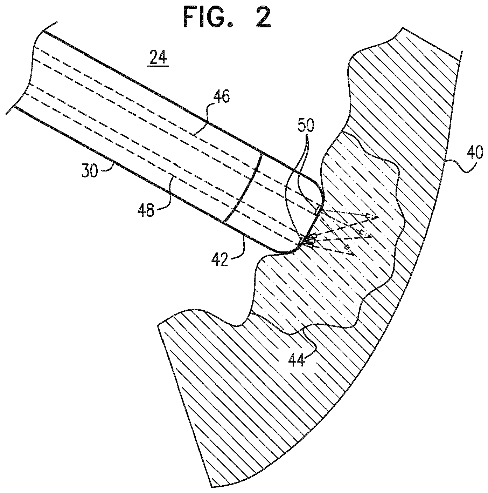

Fig. 2 is a schematic detail view showing distal end 30 of catheter 22 in contact with myocardial tissue 40 during an ablation procedure, in accordance with an embodiment of the present invention. Catheter 22 has a conductive cap 42 at its distal end. Typically, cap 42 comprises a biocompatible metal suitable to serve as an ablation electrode, such as gold, palladium, platinum, or an alloy of these materials, for example. An electrical conductor (not shown) in catheter 22 conveys electrical energy from RF generator 34, through catheter 22, to cap 42, in order to energize the cap to ablate myocardial tissue with which the cap is in contact, thus creating a lesion 44. Further details of a catheter and cap having these features are described, for example, in the above-mentioned U.S. Patent Application Publication 2014/0171936.

Catheter 22 comprises optical fibers 46, 48, which extend through the catheter between optical module 36 and respective optical ports 50 opening through cap 42 in distal end 30. In the pictured example, fiber 46 emits light into the ablation site, while fiber 48 receives the light that is scattered from the tissue and returns it to the optical module. The term "light," in the context of the present description and in the claims, refers to optical radiation in at least the visible and the infrared wavelength bands but might also include ultraviolet radiation.

Although two fibers 46, 48 and corresponding ports 50 are shown in Fig. 2, catheter 22 may alternatively comprise a smaller or large number of optical fibers, as well as light emitters and receivers of other sorts. For example, miniature light sources and detectors, such as suitable LEDs and photodiodes, may be embedded in the catheter tip in order to emit and sense received light. Additionally or alternatively, the catheter may comprise lenses and/or other types of transmission and collection optics.

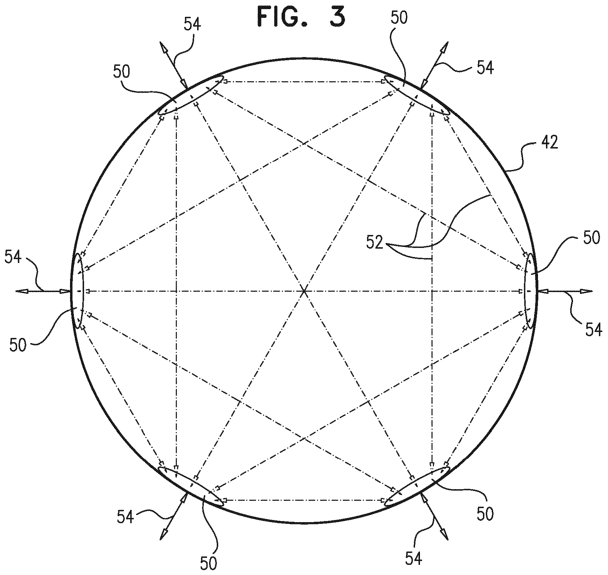

Fig. 3 is a schematic end view of cap 42 at distal end 30 of catheter 22, in accordance with an embodiment of the present invention. In this view, it is assumed that six optical fibers passing through the catheter, like fibers 46 and 48 in Fig. 2, terminate at respective windows 50 at different locations in cap 42. This arrangement enables different combinations of fibers to be used in probing different locations within lesion 44. The available probing paths include single-window paths 54, in which the light emitted from a given window 50 returns to the same window, so that the same fiber serves as emitter and receiver. Inter-window paths 52 define configurations in which light from a given window returns to a different window.

The scattered light received from any given path 52, 54 or group of paths depends on characteristics of tissue in the path or group of paths. Longer paths tend to probe deeper into tissue 40. The inventors have found that for purposes of the measurements described hereinbelow, it is useful to illuminate the tissue through one of windows 50 and receive the scattered radiation via multiple paths 52 simultaneously, for example through all of the other windows. This approach gives good coverage of the region of lesion 44 and high signal/noise ratio. Alternative, other paths and combinations of paths may be used in order to enhance spatial resolution.

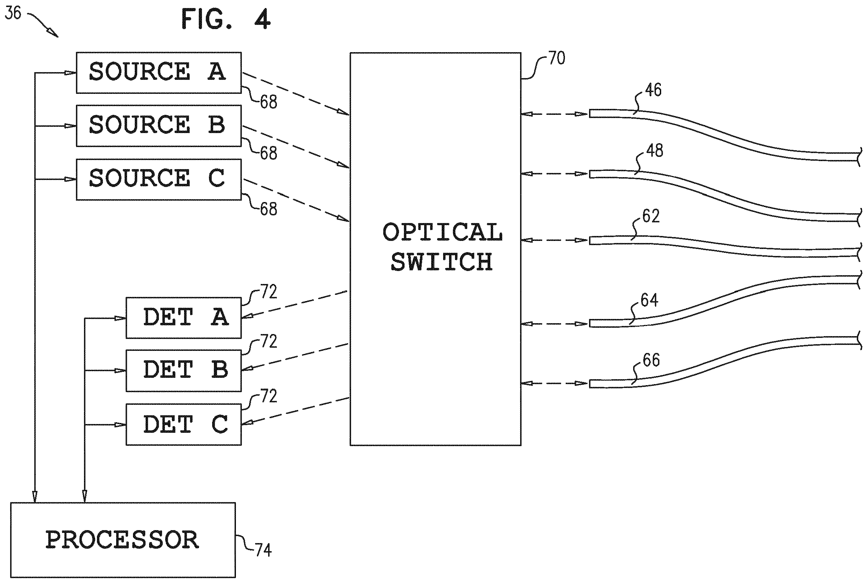

Fig. 4 is a block diagram that schematically shows details of optical module 36 in console 32, in accordance with an embodiment of the present invention. One or more radiation sources 68 emit optical radiation. An optical switch 70 is set to select one or more of optical fibers 46, 48, 62, 64, 66, ..., running through catheter 22, that are to receive the emitted radiation and transmit the radiation to the tissue at the ablation site. Switch 70 likewise routes the scattered radiation returned from the ablation site by one or more of the optical fibers to one or more detectors 72. Optical switch 70 may comprise, for example, a suitable arrangement of movable reflectors, as well as focusing elements, for directing light along the desired paths. Alternatively or additionally, switch 70 may comprise a chopper wheel and beam splitters that allow only one source at a time to couple to the fibers, while allowing the detectors to receive light from all fibers. Optionally, switch 70 may also include optical filters and/or other wavelengthselective or dispersive elements as an aid to wavelengthresolved measurements. Various designs of optical module 36 that support the measurement schemes described herein will be apparent to those skilled in the art after reading the present description, and all such designs are considered to be within the scope of the present invention.

In some embodiments, each of sources 68 comprises a narrowband optical emitter, such as a suitable LED or laser diode, operating at a particular measurement wavelength. Possible criteria for selecting these wavelengths are illustrated in the figures that follow. For example, source A may comprise a red light source, while source B comprises an infrared source. More specifically, source A typically emits light at a wavelength between 600 and 700 nm, while source B emits light at a wavelength between 700 and 800 nm. In one advantageous implementation, source A emits light at a wavelength between 630 and 670 nm, while source B emits light at a wavelength between 750 and 790 nm, and source C emits light at a third wavelength between 670 and 710 nm. The usefulness of these particular wavelength ranges in estimating lesion size is explained further hereinbelow.

Alternatively or additionally, one or more of sources 68 may comprise a broadband source, which typically emits light over a range of wavelengths in at least the infrared and visible ranges. In this case, optical switch 70 may comprise a dispersive element, such as a grating or prism, which separates the different wavelength components of the scattered light received from the ablation site among the different detectors 72, so that each detector receives a different wavelength or wavelength range, such as the red and infrared ranges mentioned above. The measurement results shown in Figs.

5 and 6 were obtained in this manner.

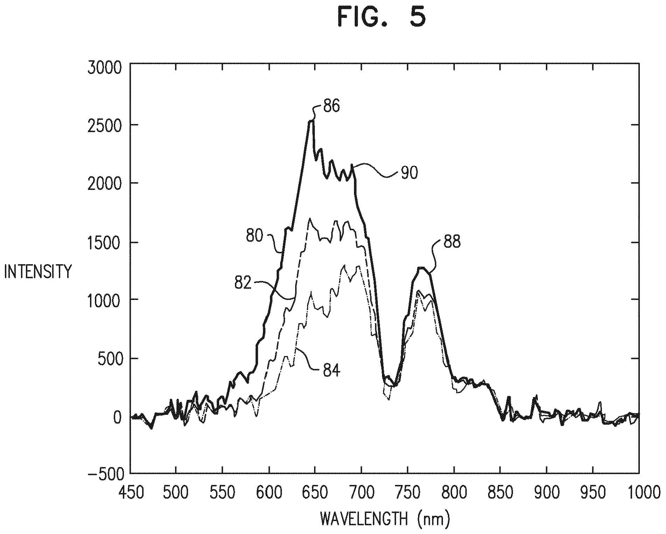

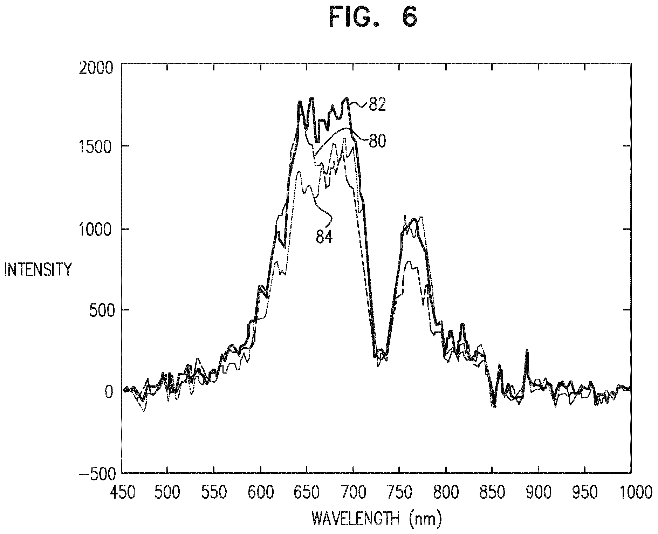

Figs. 5 and 6 are schematic spectral plots of scattered light intensity from ablation sites at successive stages in ablation procedures at the sites, in accordance with an embodiment of the present invention. The plots show spectral intensity as a function of wavelength at three different points in time during two different ablation procedures (selected from among 17 lesions created in procedures performed on experimental animals). The measurements were made using a system and catheter similar to those illustrated and described above. In each case, broadband radiation was delivered to the ablation site through one of the optical fibers, and the scattered radiation was received through one or more other fibers and measured spectroscopically.

Three spectroscopic curves are shown in each figure: a pre-ablation spectrum 80, an intermediate spectrum 82 captured during the ablation procedure, and a post-ablation spectrum 84. The spectra consistently exhibited the sort of bi-modal structure that is shown in Figs. 5 and 6, with a red band-edge peak 86 in the range of 643-650 nm and a near infrared peak 88 at 765-772 nm. The intensities at peaks 86 and 88 was measured, along with an intermediate peak 90 at the intermediate band edge located at 690-698 nm (on the border between red and infrared). In general, the intensity decreased during the ablation process over the entire spectral band of interest (between about 600 and 800 nm) and could thus give an indication of the ablation in progress.

The inventors found, however, that the ratios between the post- and pre-ablation measurements of the scattered light intensities at peaks 86 and 88 gave a more reliable estimate of the size of the lesion created by the ablation, and that comparison between these ratios - which are generally different from one another - gives a useful indication of lesion depth. The estimate is improved still further when the ratio of post- and pre-ablation measurements at peak 90 is also evaluated, and a mathematical relation is evaluated between the ratios at the three peaks.

In particular, the product of the ratios at peaks 88 and 90 divided by the ratio at peak 86 gives the overall ratio value L, which was found experimentally to increase in proportion to the lesion depth:

$$L = \frac{\left(E_2/S_2\right)\left(E_3/S_3\right)}{\left(E_1/S_1\right)}$$

In this expression, $S_j$ is the pre-ablation intensity at peak 86 ($S_1$), peak 88 ($S_2$), or peak 90 ($S_3$), while $E_j$ is the corresponding intensity at a subsequent stage of the ablation (which may be an intermediate stage or completion of the procedure). In other words, deeper ablation is characterized by a large drop in the spectral intensity at the infrared and intermediate wavelengths relative to the drop at the red wavelength. For example, for the curves shown in Fig. 5, L = 1.32, while the actual lesion depth (measured following dissection of the heart) was 3.12 mm; whereas for the curves shown in Fig. 6, $L$ = 2.01, and the measured lesion depth was 5.71 mm.

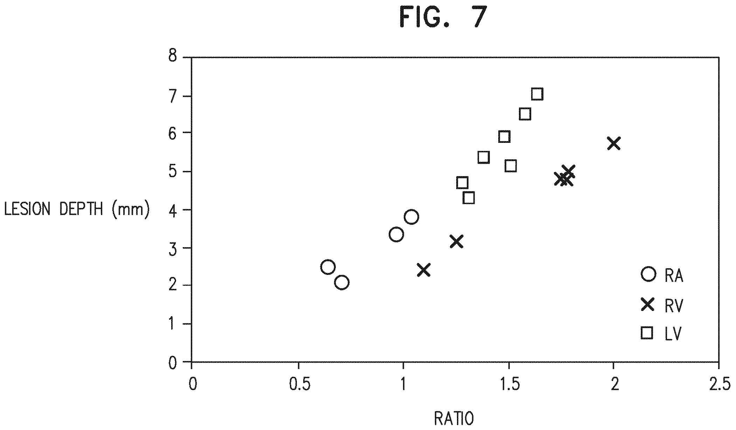

Fig. 7 is a schematic plot comparing ablation lesion depth (in millimeters) to the ratio value L over multiple lesions, in accordance with an embodiment of the present invention. The relation between L and the lesion depth varied among different chambers of the heart, and therefore, different symbols are used to indicate the results measured in the right atrium (RA), right ventricle (RV) and left ventricle (LV). In all cases, however, the lesion depth scaled clearly, in a roughly linear manner, with the ratio value L.

To apply these principles in system 20, optical module 36 measures the spectral intensity of the light scattered from the ablation site, and processor 74 computes L during and after ablation. Processor 74 typically outputs this value as an indication to operator 28. Additionally or alternatively, the processor may use the ratio value, typically in conjunction with other sensed parameters, in controlling the application of ablation energy autonomously or semi-autonomously.

Although the experimental results presented above make use of certain particular wavelength ranges, relations between spectral intensities at other choices of red and near-infrared wavelengths may be used to similar effect. It should be understood in this regard that a ratio between a pair of successive measurements or a relation between dividend and divisor may be expressed equivalently as the quotient of the first value divided by the second or the second value divided by the first. Furthermore, although ratios are used in the embodiments described above in comparing different spectral intensity values, other arithmetic operations, such as subtraction, may alternatively be applied in comparing spectral values and computing quantitative measures of change.

It will thus be appreciated that the embodiments described above are cited by way of example, and that the present invention is not limited to what has been particularly shown and described hereinabove. Rather, the scope of the present invention is defined by the appended claims.

**Claims**

1. Medical apparatus, comprising:

an invasive probe (22), which is configured to be inserted into a body of a living subject, to direct light at a plurality of different wavelengths toward a treatment site within the body and to receive the light scattered from the site;
an optical module (36), which is coupled to the invasive probe so as to make, at a first stage in ablation of tissue at the treatment site, first measurements of scattered light intensities from the site at the plurality of different wavelengths, and to make, at a second stage in the ablation of the tissue, subsequent to the first stage, second measurements of the scattered light intensities from the site at the plurality of different wavelengths, wherein the plurality of the different wavelengths comprises a first wavelength in a visible light range and a second wavelength in an infrared light range; and wherein a processor (74), which is configured to assess a progress of the ablation by computing different, respective measures of change in the scattered light intensities at the different wavelengths occurring between the first and second measurements, and comparing the respective measures.

2. The apparatus according to claim 1, wherein the probe comprises a catheter, which is configured to ablate myocardial tissue in a heart of the subject.

3. The apparatus according to claim 1, wherein the probe comprises an emitter, which is configured to direct light toward the site, and a receiver, which is configured to collect the light scattered from the tissue.

4. The apparatus according to claim 3, wherein the emitter and the receiver comprise at least one optical fiber (46, 48, 62, 64, 66), which extends through the probe between an optical port in proximity to the site at a distal end of the probe and the optical module coupled to a proximal end of the probe.

5. The apparatus according to claim 1, wherein the first wavelength is between 600 and 700 nm, and the second wavelength is between 700 and 800 nm.

6. The apparatus according to claim 5, wherein the first wavelength is between 630 and 670 nm, and the second wavelength is between 750 and 790 nm, and wherein the plurality of the different wavelengths comprises a third wavelength between 670 and 710 nm.

7. The apparatus according to claim 6, wherein the different, respective measures computed by the processor comprise first, second and third ratios between the first and second measurements of the scattered light intensities at the first, second and third wavelengths, respectively, and the processor is configured to compare the respective measures by evaluating a mathematical relation between first, second and third ratios in order to assess the progress of the ablation.

8. The apparatus according to claim 7, wherein evaluating the mathematical relation comprises estimating a size of a lesion created by the ablation at the site based on a product of the second and third ratios divided by the first ratio.

9. The apparatus according to claim 1, wherein the different, respective measures computed by the processor comprise at least first and second ratios between the first and second measurements of the scattered light intensities at the first and second wavelengths, and wherein the processor is configured to estimate a size of a lesion created by the ablation at the site based on a comparison between the first and second ratios.

**Patentansprüche**

1. Medizinisches Gerät, umfassend:

   eine invasive Sonde (22), die ausgestaltet ist, um in einen Körper eines lebenden Subjekts eingeführt zu werden, um Licht mit einer Vielzahl unterschiedlicher Wellenlängen in Richtung eines Behandlungssitus innerhalb des Körpers zu lenken und das von dem Situs gestreute Licht zu empfangen;
   ein Optikmodul (36), das an die invasive Sonde gekoppelt ist, um so in einer ersten Stufe in der Ablation von Gewebe an dem Behandlungssitus erste Messungen der Intensitäten des gestreuten Lichts von dem Situs bei der Vielzahl unterschiedlicher Wellenlängen durchzuführen und in einer zweiten Stufe in der Ablation von Gewebe, nach der ersten Stufe, zweite Messungen der Intensitäten des gestreuten Lichts von dem Situs bei der Vielzahl unterschiedlicher Wellenlängen durchzuführen, wobei die Vielzahl der unterschiedlichen Wellenlängen eine erste Wellenlänge im Bereich des sichtbaren Lichts und eine zweite Wellenlänge in einem Bereich des Infrarots umfasst; und wobei ein Prozessor (74), der konfiguriert ist, um einen Fortschritt der Ablation zu beurteilen, unterschiedliche jeweilige Maße der Veränderung der Intensitäten des gestreuten Lichts bei den unterschiedlichen Wellenlängen, die zwischen der ersten und der zweiten Messungen auftreten, berechnet und die jeweiligen Maße vergleicht.

2. Gerät nach Anspruch 1, wobei die Sonde einen Katheter umfasst, der konfiguriert ist, um Myokardgewebe in einem Herzen des Subjekts zu ablatieren.

3. Gerät nach Anspruch 1, wobei die Sonde einen Emitter, der konfiguriert ist, um Licht in Richtung des Situs zu lenken, und einen Empfänger umfasst, der konfiguriert ist, um das durch das Gewebe gestreute Licht zu sammeln.

**4.** Gerät nach Anspruch 3, wobei der Emitter und der Empfänger mindestens eine Lichtleiterfaser (46, 48, 62, 64, 66) umfassen, die sich zwischen einem optischen Port in der Nähe des Situs an einem distalen Ende der Sonde und dem Optikmodul, das an ein proximales Ende der Sonde gekoppelt ist, durch die Sonde hindurch erstreckt.

**5.** Gerät nach Anspruch 1, wobei die erste Wellenlänge zwischen 600 und 700 nm liegt und die zweite Wellenlänge zwischen 700 und 800 nm liegt.

**6.** Gerät nach Anspruch 5, wobei die erste Wellenlänge zwischen 630 und 670 nm liegt und die zweite Wellenlänge zwischen 750 und 790 nm liegt, und wobei die Vielzahl der unterschiedlichen Wellenlängen eine dritte Wellenlänge zwischen 670 und 710 nm umfasst.

**7.** Gerät nach Anspruch 6, wobei die unterschiedlichen jeweiligen Maße, die durch den Prozessor berechnet werden, erste, zweite und dritte Verhältnisse zwischen den ersten und zweiten Messungen der Intensitäten des gestreuten Lichts bei der ersten, zweiten beziehungsweise dritten Wellenlänge umfassen und der Prozessor konfiguriert ist, um die jeweiligen Maße zu vergleichen, indem eine mathematische Beziehung zwischen ersten, zweiten und dritten Verhältnissen ausgewertet wird, um den Fortschritt der Ablation zu beurteilen.

**8.** Gerät nach Anspruch 7, wobei Auswerten der mathematischen Beziehung Schätzen einer Größe einer Läsion, die durch die Ablation an dem Situs erzeugt worden ist, basierend auf einem Produkt der zweiten und dritten Verhältnisse, geteilt durch das erste Verhältnis, umfasst.

**9.** Gerät nach Anspruch 1, wobei die unterschiedlichen jeweiligen Maße, die durch den Prozessor berechnet wurden, mindestens erste und zweite Verhältnisse zwischen den ersten und zweiten Messungen der Intensitäten des gestreuten Lichts bei den ersten und zweiten Wellenlängen umfasst, und wobei der Prozessor konfiguriert ist, um eine Größe einer Läsion, die durch Ablation an dem Situs erzeugt wurde, basierend auf einem Vergleich zwischen den ersten und zweiten Verhältnissen zu schätzen.

**Revendications**

**1.** Appareil médical, comprenant :

une sonde invasive (22), qui est configurée pour être insérée dans un corps d'un sujet vivant, pour diriger de la lumière à une pluralité de longueurs d'onde différentes vers un site de traitement à l'intérieur du corps et pour recevoir la lumière diffusée depuis le site ;

un module optique (36), qui est couplé à la sonde invasive de manière à effectuer, à un premier stade de l'ablation du tissu au niveau du site de traitement, des premières mesures des intensités de la lumière diffusée à partir du site à la pluralité de différentes longueurs d'onde, et à effectuer, à un deuxième stade de l'ablation du tissu, après la première étape, des deuxièmes mesures des intensités de la lumière diffusée depuis le site à la pluralité de différentes longueurs d'onde, la pluralité des différentes longueurs d'onde comprenant une première longueur d'onde dans une plage de lumière visible et une deuxième longueur d'onde dans une plage de lumière infrarouge ; et

un processeur (74), qui est configuré pour évaluer une progression de l'ablation en calculant différentes mesures respectives de changement dans les intensités de lumière diffusée aux différentes longueurs d'onde se produisant entre les première et deuxième mesures, et en comparant les mesures respectives.

**2.** Appareil selon la revendication 1, la sonde comprenant un cathéter, qui est configuré pour effectuer une ablation du tissu myocardique dans un cœur du sujet.

**3.** Appareil selon la revendication 1, la sonde comprenant un émetteur, qui est configuré pour diriger la lumière vers le site, et un récepteur, qui est configuré pour collecter la lumière diffusée par le tissu.

**4.** Appareil selon la revendication 3, l'émetteur et le récepteur comprenant au moins une fibre optique (46, 48, 62, 64, 66), qui s'étend à travers la sonde entre un port optique à proximité du site à une extrémité distale de la sonde et le module optique couplé à une extrémité proximale de la sonde.

**5.** Appareil selon la revendication 1, la première longueur d'onde étant comprise entre 600 et 700 nm, et la deuxième longueur d'onde étant comprise entre 700 et 800 nm.

**6.** Appareil selon la revendication 5, la première longueur d'onde étant comprise entre 630 et 670 nm, et la deuxième longueur d'onde étant comprise entre 750 et 790 nm, et la pluralité des différentes longueurs d'onde comprenant une troisième longueur d'onde comprise entre 670 et 710 nm.

**7.** Appareil selon la revendication 6, les différentes mesures respectives calculées par le processeur comprenant des premier, deuxième et troisième rapports entre les première et deuxième mesures des intensités de lumière diffusée, respectivement, aux première, deuxième et troisième longueurs d'onde, et le processeur étant configuré pour comparer les mesures respectives en évaluant une relation mathématique entre les premier, deuxième et troisième rapports afin d'évaluer la progression de l'ablation.

8. Appareil selon la revendication 7, l'évaluation de la relation mathématique comprenant l'estimation de la taille d'une lésion créée par l'ablation au niveau du site sur la base d'un produit des deuxième et troisième rapports divisé par le premier rapport.

9. Appareil selon la revendication 1, les différentes mesures respectives calculées par le processeur comprenant au moins des premier et deuxième rapports entre les première et deuxième mesures des intensités de lumière diffusée aux première et deuxième longueurs d'onde, et le processeur étant configuré pour estimer une taille d'une lésion créée par l'ablation au niveau du site sur la base d'une comparaison entre les premier et deuxième rapports.

FIG. 1

EP 3 040 044 B1

# FIG. 2

# FIG. 3

FIG. 4

# FIG. 5

EP 3 040 044 B1

# FIG. 6

EP 3 040 044 B1

FIG. 7

EP 3 040 044 B1

**EP 3 040 044 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7001383 B
- US 20140171936
- US 8147484 B
- US 20120265184 A1
- EP 2742892 A1